# EUROPEAN PATENT APPLICATION

(11) **EP 2 110 029 A1**
(43) Date of publication of application: **21.10.2009**
(21) Application number: 08007424.8
(22) Date of filing: 16.04.2008
(51) Int. Cl.: A23L 1/305

(54) **Supplement compositions for protein-based diets**

(71) Applicant: Italfarmacia S.r.l., 00155 Roma (IT)
(72) Inventor: Cavallo, Giovanni, 00122 Ostia (Roma) (IT); Piccioli, Piero, 00162 Roma (IT); Marchetti, Mario, 00161 Roma (IT)
(74) Representative: Celestino, Marco

(57) **Abstract**

A human use supplement composition that prevents disadvantages of protein-based diets, such as constipation, meteorism and flatulence, bad breath, It is also suitable for diabetes and other blood sugar-related illnesses affected patients and is helpful to prevent and cure diabetic complications. The composition contains a milk protein, an amino acid, a dietary component selected among a polysaccharide, and enzyme or a combination thereof. The polysaccharide has a polymer chain composed principally of fructose units linked together by β(2→1) glycosidic bonds at the anomeric C2. The degree of polymerisation of the polysaccharide is the number of monomer units in the polymer chain. The enzyme is selected among α-Galactosidase; Lactase, a mixture of α-Galactosidase and Lactase, another enzyme having a flatulence reducing action.

## Description

### DESCRIPTION

### Field of the invention.

The present invention relates to human use supplement compositions for protein-based diets, said supplements being able to compensate for said diets typical drawbacks.

### Background of the invention.

A complete and balanced diet is compulsory to maintain good health. It is fundamental to supply the body with correct quantities of all necessary nutrients: proteins, lipids, sugars, vitamins and minerals.

Proteins are composed by amino acids. The human organism is able to synthesize most of the amino acids by itself, with the exception of nine, called essential amino acids, which therefore have to be taken with the diet.

In particular, the essential amino acids (Leucine, Lysine, Isoleucine, Valine, Phenilalanine, Threonine, Methionine, Histidine and Tryptophan) are important to build up the muscles. Besides, these essential amino acids should be in a defined ratio between them to perform their highest plastic action.

A protein-based diet is therefore highly recommended in those cases where said plastic action can play an important role. For instance, in the case of sports practice, which needs a wide and strong muscular solicitation, it is advisable to adopt a protein rich alimentary regimen.

Another field where protein-based diets are widely used is the cure and prevention of obesity, to cope with the risk of muscle mass loss.

Protein-based diets to cure obesity are normally composed by a part constituted by a soluble dietary protein supplement comprising amino acids in recommended proportions and by a part constituted by protein foods at low glucidic content, as in the case of meat and fish. Typically, each of said parts can build up 50% of the diet.

A diet with low calories, based only on proteins intake, is able to reduce insulin in such extent that lipogenesis is hindered and in the same time lipolysis is stimulated, saving in the meanwhile the metabolic activity in noble tissues and assuring an equilibrated balance of nitrogen.

More in depth, the mechanism by which a protein-based diet against obesity operates is the following: ingestion of essential amino acids in correct proportions stimulates the secretion of Growth Hormone, with reduction of insulin. This induces the phosphorylation of the receptor of activation for the peroxisome proliferation, inhibiting the transcriptase for the adipogenesis, stimulated by the estrogens. Subsequently the mobilization of the fat from the hormone dependent regions (for example, women's flanks and men's abdomen) is enhanced, without loss of lean mass. Furthermore, individuals with low serum levels of Growth Hormone tend to develop obesity, that therefore may be fought with treatments based on or able to stimulate Growth Hormone.

A diet based on proteins, however, involves some well known drawbacks, the most common of them being intestinal disorders, in particular:
- constipation,
- meteorism and flatulence, caused above all by the lack of vegetable fibres, and
- bad breath, caused by the process of ketosis, when fat is broken down by the body.
   As known, there is a strict association between obesity and the probability to develop type 2 or "adult onset" diabetes mellitus, the most common form of diabetes mellitus.
   Diabetes mellitus is a disease characterised by impaired glucose metabolism that leads to angiopathy, in particular to diabetic microangiopathy, known manifestations of said diabetic microangiopathy being diabetic retinopathy, brain micro-infarcts and diabetic nephropathy.
   A protein-based regimen is normally considered an useful aid in diabetes treatment. Nevertheless, according to more recent investigations (Karabatas et al., P.S.E.B.M. 2000, Vol. 224: 159-165), unwanted long term side effects are possible: in particular, in a diabetic patient renal functionality may be reduced (an evidence of this can be high Glomerular Filtration Rate or GFR values, i.e. the volume of fluid filtered from the kidney glomerular capillaries per unit time, a parameter normally measured to determine renal function); a diet rich in proteins may therefore lead to deficiencies in elimination of final metabolites resulting from protein catabolism and consequently to kidney hypertrophy, a possible cause of diabetic nephropathy.

### Summary of the invention

It is then a feature of the present invention to provide a human use supplement composition for protein-based diets which prevents constipation, a typical disadvantage of protein-based diets.

Another feature of the present invention is to provide a human use supplement composition for protein-based diets which prevents meteorism and flatulence, other typical disadvantages of protein-based diets.

It also a feature of the present invention to provide a human use supplement composition for protein-based diets which prevents together meteorism, flatulence and constipation.

It is also a feature of the present invention to provide a human use supplement composition for protein-based diets which prevents bad breath, a further disadvantage of protein-based diets.

An additional feature of the present invention is to provide a human use supplement composition for protein-based diets which is suitable for diabetes and other blood sugar-related illnesses affected patients.

It is a further feature of the present invention to provide a human use supplement composition for protein-based diets which is helpful to prevent and cure diabetic complications.

These and other features are accomplished with a composition containing
- a milk protein,
- an amino acid,
   the main characteristic of said composition being that it contains also a dietary component selected from the group comprised of:

- a polysaccharide, said polysaccharide having a polymer chain, said polymer chain being composed principally of fructose units linked together by β(2→1) glycosidic bonds at the anomeric C2, said polysaccharide being characterised by a degree of polymerisation, said degree of polymerisation being the number of monomer units in the polymer chain,
- an enzyme, selected from the group comprised of
- α-Galactosidase
- Lactase,
- a mixture of α-Galactosidase and Lactase,
- an enzyme having a flatulence reducing action;
- a combination thereof.

In particular, said supplement is either in powder form or in beverage form.

Advantageously, said milk protein is selected from the group comprised of:
- α(s1) Casein,
- α(s2) Casein,
- β-Casein,
- k-Casein
- β-Lactoglobulin,
- α-Lactalbumin,
- bovine serum Albumin,
- immunoglobulins
- a combination thereof.

Advantageously, said amino acid is selected from the group comprised of:
- L-Ornithine,
- Potassium Aspartate,
- L-Taurine,
- Tryptophan,
- L-Isoleucine,
- Citrulline
- Aspartame
- Glicine
- L-Arginine
- a combination thereof.

In particular, said polysaccharide has a terminal glucose unit.

Preferably, said composition comprises a polysaccharide of natural origin, also referred to as a natural polysaccharide.

Natural polysaccharides are well known for their capacity to stimulate intestinal functionality. They acts as other soluble dietary fiber, shortening stool transit time and slightly increasing faecal bulk.

As a fermentable fiber, when metabolised by gut flora, natural polysaccharides yield short-chain fatty acids, thus producing well known favourable effects. In particular, the absorption of calcium and magnesium is enhanced, as well as the proliferation of colonic bacteria beneficial for intestinal and general health, typically Bifidobacteria and Lactobacilli (probiotic activity).

Natural polysaccharides taste from bland to slightly sweet and can advantageously be used to replace sugar, fat, and flour. Actually, the caloric content of natural polysaccharides is about 25 to 30% the food energy of sugar and other carbohydrates and ranges from one-sixth to one-ninth the caloric content of fat.

Natural polysaccharides have a minimal impact on blood sugar, therefore they are suitable for diabetics.

Said natural polysaccharide may be for example a fructooligosaccharide, also indicated with FOS, in particular said FOS has a degree of polymerisation comprised between 3 and 10.

Said natural polysaccharide may be an inulin, said inulin having a degree of polymerisation greater than 20.

Fructooligosaccharides are very soluble in water because of their low degree of polymerization. They are therefore particularly well suited to produce a protein supplement in the form of a beverage.

Inulins have a greater degree of polymerization and are therefore less soluble in water than FOS, this feature making inulins better suited to produce a protein supplement in powder form. At any rate, inulins show a solubility that increases with temperature, said solubility making allowance to use inulins to obtain a protein supplement in the form of a beverage, in particular if haze or cloudiness can be tolerated.

In particular, as above defined, said enzyme is selected from the group comprised of
- α-Galactosidase
- Lactase,
- a combination thereof.

In particular, α-Galactosidase allows the hydrolysis of some polysaccharides (α-Galactosides) that cannot otherwise be digested in the small intestine and moves in an undigested state to the large intestine, where they are fermented, hence giving rise to gas and associated discomfort. In particular, this is the case of Raffinose, a trisaccharide which is found in such aliments as beans, cabbage, Brussels sprouts, broccoli, asparagus, other vegetables, and whole grains. Said Raffinose is hydrolysed by α-Galactosidase to D-Galactose and Sucrose, which on the contrary can be easily digested. Other polysaccharides that are advantageously treated with α-Galactosidase are Stachyose (a polysaccharide of soya bean) and Melibiosc.

In a similar way, Lactase, a β-Galactosidase, is involved in the hydrolysis of Lactose (a disaccharide, in particular a β-galactoside) into constituent Galactose and Glucose. Deficiency or lack of this enzyme is the cause of the well known Lactose intolerance. Other β-Galactosidase can als be, alternatively used, as they are capable of having a flatulence reducing action

The compositions according to the invention comprise L-Arginine, free or as a constituent of milk proteins. As shown in recent investigations on rats (Reyes et al., Proc Soc.Exp.Biol.Med., June 1994, 206(2):157-61), L-Arginine administration can prevent kidney hypertrophy, said hypertrophy being due to a protein rich regimen. This effect was associated with less excretion of orotic acid in the urine. Kidney hypertrophy is to be regarded as an incipient state of diabetic nephropathy, a diabetic complication which makes it problematic to adopt protein rich diets in the case of diabetic persons.

Moreover, the compositions according to the invention comprise Glycine, free or as a constituent of milk proteins, and L-Taurine. Investigations [Alvarado-Vásquez et al., Life Sci. 13 Jun 2006, 79(3):225-32, Trachtman et al., Am J Physiol. Sep 1995, 269 (3 Pt 2):F429-38] have been performed on diabetic rats to establish the effectiveness of these amino acids in preventing and curing diabetic microangiopathy-related diseases. In the case of Glycine, the results gave evidence of a reduced incidence of opacity in lens and microaneurysms in the eyes, as well as a diminished expression of O-acetyl sialic acid in brain vessels compared with untreated diabetic rats. A less intense corporal weight loss in comparison with non-treated animals was also revealed. These results suggest that administration of Glycine attenuates the diabetic complications, probably due to inhibition of the non-enzymatic glycation process.

In the case of L-Taurine, beneficial effects on renal functionality were observed, probably related to reduced renal oxidant injury with decreased lipid peroxidation and less accumulation of AGEs within the kidney.

The compositions according to the invention can therefore provide a contribute in managing diabetic complications.

In particular, the composition contains an Inulin in the range from 125 mg to 700 mg per gram milk proteins plus amino acids.

In particular, said composition contains a FOS in the range from 125 mg to 700 mg per gram milk proteins plus amino acids.

In particular, said composition contains α-Galactosidase in the range from 6 to 700 units per gram milk proteins plus amino acids.

In particular, said composition contains lactase in the range from 3 to 350 units per gram milk proteins plus amino acids.

The only important side effect of Inulin and FOS, as well as of other constipation reducing polysaccharides, is flatulence, for they undergo the same decomposition mechanism of other polysaccharides that cannot be digested before the large intestine, hence the utility of specific anti-flatulence enzymes in a protein supplement composition containing inulin and FOS. This is the case, for instance, of endo-inulinase. In other words, anti-flatulence enzymes and anti-constipation polysaccharides have synergetic effects. Besides, compositions containing dietary components selected from both groups are to be preferred because constipation and flatulence, as protein diet associated drawbacks, usually take place together.

### Description of preferred embodiments

The composition according to the present invention will be made clearer with the not limitative following

### examples.

Eighteen examples of supplement formulations have been tested, each of said formulations being based on a composition according to the invention are shown in tables 2 to 5, said formulations comprising:
- milk proteins, a typical amino acid profile of said milk proteins being presented in Table 1. While the qualitative composition of amino acids is always defined, the percentage of each component may change (±25%), said change depending upon the whey proteins production process, in particular upon the performances reached in extraction and filtration phases.
- a combination of amino acids, said amino acids being L-Ornithine, Potassium Aspartate, L-Taurine, Tryptophan, L-Isoleucine, Citrulline, Aspartame, according to a defined ratio.

In particular,
- Table 2 presents three powder proteins - amino acids - Inulin (PAIP) formulations, the only difference being the amount of Inulin in a common protein-amino acids basic formulation, said amount ranging from about 200 mg to 665 mg per gram milk proteins plus amino acids;
- Table 3 presents six powder proteins - amino acids - enzymes (PAEP) formulations, said enzymes being α-Galactosidase and Lactase according to the ratio 2:1 in units, the only difference being the total amount of said enzymes in a common protein-amino acids basic formulation, said amount ranging from 10 to 1000 units per gram milk proteins plus amino acids;
- Table 4 presents three beverage proteins - amino acids - FOS (PAFB) formulations, the only difference being the amount of Inulin in a common protein-amino acids basic formulation, said amount ranging from about 200 mg to 665 mg per gram milk proteins plus amino acids;
- Table 5 presents six beverage proteins - amino acids - enzymes (PAEB) formulations, said enzymes being α-Galactosidase and Lactase according to the ratio 2:1 in units, the only difference being the total amount of said enzymes in a common protein - amino acids basic formulation, said amount ranging from 10 to 1000 units per gram milk-proteins plus amino acids.

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

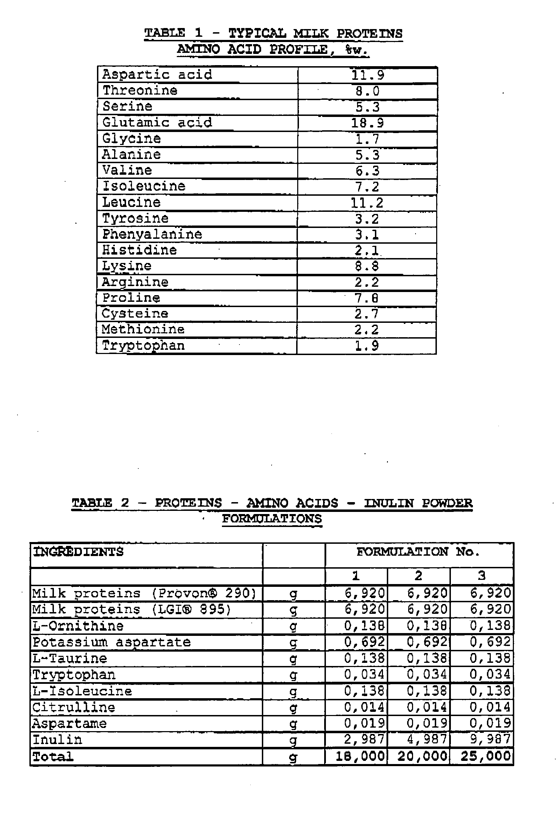

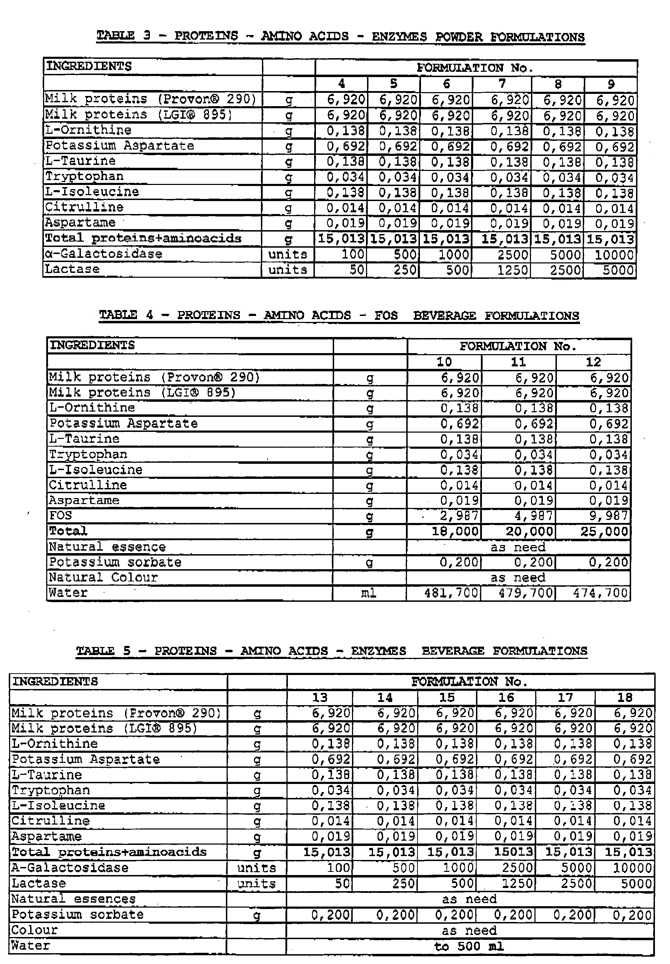

## Claims

1. A human use supplement composition for protein-based diets containing
- a milk protein,
- an amino acid
- a milk protein,
- an amino acid,
the main characteristic of said composition being that it contains also a dietary component selected from the group comprised of:
- a polysaccharide, said polysaccharide having a polymer chain, said polymer chain being composed principally of fructose units linked together by β(2→1) glycosidic bonds at the anomeric C2, said polysaccharide being **characterised by** a degree of polymerisation, said degree of polymerisation being the number of monomer units in the polymer chain;
- an enzyme, selected from the group comprised of
- α-Galactosidase
- Lactase,
- a mixture of α-Galactosidase and Lactase,
- an enzyme having a flatulence reducing action;
- a combination thereof.

2. A human use supplement composition for protein-based diets according to claim 1, wherein said supplement is either in powder form or in beverage form.

3. A human use supplement composition for protein-based diets according to claim 1, wherein said milk protein is selected from the group comprised of:
- α(s1)Casein,
- α(s2)Casein,
- β-Casein,
- k-Casein
- β-Lactoglobulin,
- α-Lactalbumin,
- bovine serum Albumin,
- immunoglobulins,
- a combination thereof.

4. A human use supplement composition for protein-based diets according to claim 1, wherein said amino acid is selected from the group comprised of:
- L-Ornithine,
- Potassium Aspartate,
- L-Taurine,
- Tryptophan,
- L-Isoleucine,
- Citrulline
- Aspartame
- Glycine
- L-Arginine
- a combination thereof

5. A human use supplement composition for protein-based diets according to claim 1, wherein said polysaccharide has a terminal glucose unit.

6. A human use supplement composition for protein-based diets according to claim 1, wherein said composition comprises a polysaccharide of natural origin.

7. A human use supplement composition for protein-based diets according to claim 6, wherein said natural polysaccharide is an inulin, said inulin having a degree of polymerisation greater than 20.

8. A human use supplement composition for protein-based diets according to claim 1, wherein said polysaccharide is a fructooligosaccharide, also indicated with FOS

9. A human use supplement composition for protein-based diets according to claim 8, wherein said FOS has a degree of polymerisation comprised between 3 and 10.

10. A human use supplement composition for protein-based diets according to claim 1, wherein said composition comprises milk proteins contatining L-Arginine as an amino acid constituent.

11. A human use supplement composition for protein-based diets according to claim 1, wherein said composition comprises milk proteins contatining L-Taurine as an amino acid constituent.

12. A human use supplement composition for protein-based diets according to claim 1, wherein said composition contains an inulin in the range from 125 mg to 700 mg per gram milk proteins plus amino acids.

13. A human use supplement composition for protein-based diets according to claim 1, wherein said composition contains a FOS in the range from 125 mg to 700 mg per gram milk proteins plus amino acids.

14. A human use supplement composition for protein-based diets according to claim 1, wherein said composition contains α-Galactosidase in the range from 6 to 700 units per gram milk proteins plus amino acids.

15. A human use supplement composition for protein-based diets according to claim 1, wherein said composition contains Lactase in the range from 3 to 350 units per gram milk proteins plus amino acids.
